# EUROPEAN PATENT SPECIFICATION

(11) **EP 2 895 862 B1**
(45) Date of publication and mention of the grant of the patent: **09.11.2016**
(21) Application number: 13780187.4
(22) Date of filing: 12.09.2013
(51) Int. Cl.: G01N 33/569

(54) **BIOMARKER**
BIOMARKER
BIOMARQUEUR

(30) Priority: 17.09.2012 GB 201216562
(43) Date of publication of application: 22.07.2015
(73) Proprietor: Oxford University Innovation Limited, Oxford OX2 0SZ (GB)
(72) Inventor: CASALS-PASCUAL, Climent, Headington Oxford Oxfordshire OX3 7BN (GB)
(74) Representative: Atkinson, Jennifer
(86) International application number: PCT/GB2013/052391
(87) International publication number: WO 2014/041356

(56) References cited:
- HONGLEI HUANG ET AL: "Proteomic identification of host and parasite biomarkers in saliva from patients with uncomplicated Plasmodium falciparum malaria", MALARIA JOURNAL, BIOMED CENTRAL, LONDON, GB, vol. 11, no. 1, 28 May 2012 (2012-05-28), page 178, XP021126680, ISSN: 1475-2875, DOI: 10.1186/1475-2875-11-178
- Abba K ET AL: "Rapid diagnostic tests for diagnosing uncomplicated P. falciparum malaria in endemic countries (Review) Rapid diagnostic tests for diagnosing uncomplicated P. falciparum malaria in endemic countries (Review)", , 6 July 2011 (2011-07-06), XP055094929, Retrieved from the Internet: URL:http://onlinelibrary.wiley.com/store/1 0.1002/14651858.CD008122.pub2/asset/CD0081 22.pdf?v=1&t=hq4xhajc&s=1efc7d7fd2417fd64d 299ccc7bd60868c139322c [retrieved on 2014-01-07]
- C Unknown: "Coomassie Brilliant Blue R-250", , 3 September 2012 (2012-09-03), XP055094916, Retrieved from the Internet: URL:http://en.wikipedia.org/w/index.php?ti tle=Coomassie_Brilliant_Blue&oldid=5106115 32 [retrieved on 2014-01-07]
- SHAHABUDDIN M ET AL: "The gene for hypoxanthine phosphoribosyl transferase of Plasmodium falciparum complements a bacterial HPT mutation", MOLECULAR AND BIOCHEMICAL PARASITOLOGY, ELSEVIER SCIENCE PUBLISHERS, AMSTERDAM, NL, vol. 41, no. 2, 1 July 1990 (1990-07-01), pages 281-288, XP023700174, ISSN: 0166-6851, DOI: 10.1016/0166-6851(90)90191-N [retrieved on 1990-07-01]
- MARIE L. THÉZÉNAS ET AL: "PfHPRT: A New Biomarker Candidate of Acute Plasmodium falciparum Infection", JOURNAL OF PROTEOME RESEARCH, vol. 12, no. 3, 1 March 2013 (2013-03-01), pages 1211-1222, XP055094821, ISSN: 1535-3893, DOI: 10.1021/pr300858g

### SEQUENCE LISTING

<110> Isis Innovation Limited
<120> Biomarker
<130> JA64249P.WOP
<150> GB1216562.7
   <151> 2012-09-17
<160> 102
<170> PatentIn version 3.5
<210> 1
   <211> 250
   <212> PRT
   <213> Plasmodium falciparum
<400> 1
   Met Thr Thr Tyr Thr Leu Val Leu Leu Arg His Gly Glu Ser Thr Trp
   1               5                   10                  15
   Asn Lys Glu Asn Lys Phe Thr Gly Trp Thr Asp Val Pro Leu Ser Glu
               20                  25                  30
   Lys Gly Glu Glu Glu Ala Ile Ala Ala Gly Lys Tyr Leu Lys Glu Lys
           35                  40                  45
   Asn Phe Lys Phe Asp Val Val Tyr Thr Ser Val Leu Lys Arg Ala Ile
       50                  55                  60
   Cys Thr Ala Trp Asn Val Leu Lys Thr Ala Asp Leu Leu His Val Pro
   65                  70                  75                  80
   Val Val Lys Thr Trp Arg Leu Asn Glu Arg His Tyr Gly Ser Leu Gln
                   85                  90                  95
   Gly Leu Asn Lys Ser Glu Thr Ala Lys Lys Tyr Gly Glu Glu Gln Val
               100                 105                 110
   Lys Ile Trp Arg Arg Ser Tyr Asp Ile Pro Pro Pro Lys Leu Asp Lys
           115                 120                 125
   Glu Asp Asn Arg Trp Pro Gly His Asn Val Val Tyr Lys Asn Val Pro
       130                 135                 140
   Lys Asp Ala Leu Pro Phe Thr Glu Cys Leu Lys Asp Thr Val Glu Arg
   145                 150                 155                 160
   Val Leu Pro Phe Trp Phe Asp His Ile Ala Pro Asp Ile Leu Ala Asn
                   165                 170                 175
   Lys Lys Val Met Val Ala Ala His Gly Asn Ser Leu Arg Gly Leu Val
               180                 185                 190
   Lys His Leu Asp Asn Leu Ser Glu Ala Asp Val Leu Glu Leu Asn Ile
           195                 200                 205
   Pro Thr Gly Val Pro Leu Val Tyr Glu Leu Asp Glu Asn Leu Lys Pro
       210                 215                 220
   Ile Lys His Tyr Tyr Leu Leu Asp Ser Glu Glu Leu Lys Lys Lys Met
   225                 230                 235                 240
   Asp Glu Val Ala Asn Gln Gly Lys Ala Lys
                   245                 250
<210> 2
   <211> 231
   <212> PRT
   <213> Plasmodium falciparum
<400> 2
   Met Pro Ile Pro Asn Asn Pro Gly Ala Gly Glu Asn Ala Phe Asp Pro
   1               5                   10                  15
   Val Phe Val Lys Asp Asp Asp Gly Tyr Asp Leu Asp Ser Phe Met Ile
               20                  25                  30
   Pro Ala His Tyr Lys Lys Tyr Leu Thr Lys Val Leu Val Pro Asn Gly
           35                  40                  45
   Val Ile Lys Asn Arg Ile Glu Lys Leu Ala Tyr Asp Ile Lys Lys Val
       50                  55                  60
   Tyr Asn Asn Glu Glu Phe His Ile Leu Cys Leu Leu Lys Gly Ser Arg
   65                  70                  75                  80
   Gly Phe Phe Thr Ala Leu Leu Lys His Leu Ser Arg Ile His Asn Tyr
                   85                  90                  95
   Ser Ala Val Glu Thr Ser Lys Pro Leu Phe Gly Glu His Tyr Val Arg
               100                 105                 110
   Val Lys Ser Tyr Cys Asn Asp Gln Ser Thr Gly Thr Leu Glu Ile Val
           115                 120                 125
   Ser Glu Asp Leu Ser Cys Leu Lys Gly Lys His Val Leu Ile Val Glu
       130                 135                 140
   Asp Ile Ile Asp Thr Gly Lys Thr Leu Val Lys Phe Cys Glu Tyr Leu
   145                 150                 155                 160
   Lys Lys Phe Glu Ile Lys Thr Val Ala Ile Ala Cys Leu Phe Ile Lys
                   165                 170                 175
   Arg Thr Pro Leu Trp Asn Gly Phe Lys Ala Asp Phe Val Gly Phe Ser
               180                 185                 190
   Ile Pro Asp His Phe Val Val Gly Tyr Ser Leu Asp Tyr Asn Glu Ile
           195                 200                 205
   Phe Arg Asp Leu Asp His Cys Cys Leu Val Asn Asp Glu Gly Lys Lys
       210                 215                 220
   Lys Tyr Lys Ala Thr Ser Leu
   225                 230
<210> 3
   <211> 54
   <212> PRT
   <213> Plasmodium falciparum
<400> 3
   Phe Thr Gly Trp Thr Asp Val Pro Leu Ser Glu Lys Gly Glu Glu Glu
   1               5                   10                  15
   Ala Ile Ala Ala Gly Lys Ala Ile Cys Thr Ala Trp Asn Val Leu Lys
               20                  25                  30
   His Tyr Gly Ser Leu Gln Gly Leu Asn Lys His Tyr Tyr Leu Leu Asp
           35                  40                  45
   Ser Glu Glu Leu Lys Lys
       50
<210> 4
   <211> 54
   <212> PRT
   <213> Plasmodium vivax
<400> 4
   Phe Thr Gly Trp Thr Asp Val Pro Leu Ser Glu Gln Gly Glu Gln Glu
   1               5                   10                  15
   Ala Ile Ala Ala Gly Asn Ala Ile Thr Thr Thr Trp Asn Val Leu Lys
               20                  25                  30
   His Tyr Gly Ser Leu Gln Gly Leu Asn Lys His Tyr Tyr Leu Leu Asp
           35                  40                  45
   Ser Glu Glu Leu Lys Lys
       50
<210> 5
   <211> 54
   <212> PRT
   <213> Plasmodium knowlesi
<400> 5
   Phe Thr Gly Trp Thr Asp Val Pro Leu Ser Glu Gln Gly Glu Lys Glu
   1 5 10 15
   Ala Ile Ser Ala Gly Asn Ala Ile Thr Thr Thr Trp Asn Val Leu Lys
   20 25 30
   His Tyr Gly Ser Leu Gln Gly Leu Asn Lys His Tyr Tyr Leu Met Asp
   35 40 45
   Ser Glu Glu Leu Lys Lys
   50
<210> 6
   <211> 55
   <212> PRT
   <213> Homo sapiens
<400> 6
   Phe Ser Gly Trp Tyr Asp Ala Asp Leu Ser Gln Ala Gly His Glu Glu
   1 5 10 15
   Ala Lys Arg Gly Gly Gln Ala Ile Arg Thr Leu Trp Thr Val Leu Asp
   20 25 30
   His Tyr Gly Gly Leu Thr Gly Leu Asn Lys Pro Met Gln Phe Leu Gly
   35 40 45
   Asp Glu Glu Thr Val Arg Lys
   50 55
<210> 7
   <211> 49
   <212> PRT
   <213> Plasmodium falciparum
<400> 7
   Val Leu Val Pro Asn Gly Val Ile Lys Gly Phe Phe Thr Ala Leu Leu
   1 5 10 15
   Lys His Val Leu Ile Val Glu Asp Ile Ile Asp Thr Gly Lys Thr Val
   20 25 30
   Ala Ile Ala Cys Leu Phe Ile Lys Arg Thr Pro Leu Trp Asn Gly Phe
   35 40 45
   Lys
<210> 8
   <211> 49
   <212> PRT
   <213> Plasmodium vivax
<400> 8
   Val Leu Ile Pro Asn Gly Val Leu Lys Gly Phe Phe Ser Ala Leu Leu
   1               5                   10                  15
   Lys His Val Leu Ile Val Glu Asp Ile Ile Asp Thr Gly Lys Thr Ile
               20                  25                  30
   Ala Ile Thr Cys Leu Phe Ile Lys Arg Thr Pro Leu Trp Asn Gly Phe
           35                  40                  45
   Lys
<210> 9
   <211> 49
   <212> PRT
   <213> Plasmodium knowlesi
<220>
   <221> misc_feature
   <222> (49) .. (49)
   <223> Xaa can be any naturally occurring amino acid
<400> 9
   Val Leu Ile Pro Asn Gly Val Leu Arg Gly Phe Phe Ser Ala Leu Leu
   1               5                   10                  15
   Lys His Val Leu Ile Val Glu Asp Ile Ile Asp Thr Gly Lys Thr Ile
               20                  25                  30
   Ala Ile Thr Cys Leu Phe Ile Lys Arg Thr Pro Leu Trp Asn Gly Phe
           35                  40                  45
   Xaa
<210> 10
   <211> 49
   <212> PRT
   <213> Homo sapiens
<400> 10
   Val Phe Ile Pro His Gly Leu Ile Met Lys Phe Phe Ala Asp Leu Leu
   1               5                   10                  15
   Asp Asn Val Leu Ile Val Glu Asp Ile Ile Asp Thr Gly Lys Met Val
               20                  25                  30
   Lys Val Ala Ser Leu Leu Val Lys Arg Thr Pro Arg Ser Val Gly Tyr
           35                  40                  45
   Lys
<210> 11
   <211> 7
   <212> PRT
   <213> Plasmodium falciparum
<400> 11
   Val Thr Ala Leu Asn Glu Lys
   1               5
<210> 12
   <211> 11
   <212> PRT
   <213> Plasmodium falciparum
<400> 12
   Trp Asp Asp Ile Gly Gly Leu Asp Glu Val Lys
   1               5                   10
<210> 13
   <211> 8
   <212> PRT
   <213> Plasmodium falciparum
<400> 13
   Lys Ile His Asn Asn Ile Asn Lys
   1               5
<210> 14
   <211> 10
   <212> PRT
   <213> Plasmodium falciparum
<400> 14
   Asp Ser Thr Leu Ile Met Gln Leu Leu Arg
   1               5                   10
<210> 15
   <211> 8
   <212> PRT
   <213> Plasmodium falciparum
<400> 15
   Asn Leu Leu Ser Val Ala Tyr Lys
   1               5
<210> 16
   <211> 7
   <212> PRT
   <213> Plasmodium falciparum
<400> 16
   Lys Asp Phe Glu Thr Leu Lys
   1               5
<210> 17
   <211> 11
   <212> PRT
   <213> Plasmodium falciparum
<400> 17
   Lys His Ser Glu Phe Ile Ser Phe Pro Ile Lys
   1               5                   10
<210> 18
   <211> 10
   <212> PRT
   <213> Plasmodium falciparum
<400> 18
   Gly Ser Phe Gly Glu Val Ile Leu Ser Arg
   1               5                   10
<210> 19
   <211> 16
   <212> PRT
   <213> Plasmodium falciparum
<400> 19
   Ala Thr Ala Gly Asp Thr His Leu Gly Gly Glu Asp Phe Asp Asn Arg
   1               5                   10                  15
<210> 20
   <211> 13
   <212> PRT
   <213> Plasmodium falciparum
<400> 20
   Thr Thr Pro Ser Tyr Val Ala Phe Thr Asp Thr Glu Arg
   1               5                   10
<210> 21
   <211> 11
   <212> PRT
   <213> Plasmodium falciparum
<400> 21
   Gly Ala Asp Ile Ile Val Ala Thr Pro Gly Arg
   1 5 10
<210> 22
   <211> 9
   <212> PRT
   <213> Plasmodium falciparum
<400> 22
   Asn Ala Ile Tyr His Ala Thr Phe Arg
   1 5
<210> 23
   <211> 8
   <212> PRT
   <213> Plasmodium falciparum
<400> 23
   Ile Tyr Ile His Gly Thr Gln Lys
   1 5
<210> 24
   <211> 7
   <212> PRT
   <213> Plasmodium falciparum
<400> 24
   Glu Asp Thr Ile Ser Glu Lys
   1 5
<210> 25
   <211> 18
   <212> PRT
   <213> Plasmodium falciparum
<400> 25
   Ala Ala Val Pro Ser Gly Ala Ser Thr Gly Ile Tyr Glu Ala Leu Glu
   1 5 10 15
   Leu Arg
<210> 26
   <211> 14
   <212> PRT
   <213> Plasmodium falciparum
<400> 26
   Ile Asn Pro His Val Leu Gly Glu Ile Ala Gly Ile Thr Lys
   1 5 10
<210> 27
   <211> 15
   <212> PRT
   <213> Plasmodium falciparum
<400> 27
   Asn Ile Asn Asp Leu Gln Leu Gln Thr Ser Thr Gln Ser Leu Lys
   1 5 10 15
<210> 28
   <211> 9
   <212> PRT
   <213> Plasmodium falciparum
<400> 28
   Val Val Glu Ser Ala Asp Ala Trp Arg
   1 5
<210> 29
   <211> 11
   <212> PRT
   <213> Plasmodium falciparum
<400> 29
   Tyr Glu Thr Gly Gly Thr Leu Glu Gly Leu Arg
   1 5 10
<210> 30
   <211> 10
   <212> PRT
   <213> Plasmodium falciparum
<400> 30
   Ala Ile Cys Thr Ala Trp Asn Val Leu Lys
   1 5 10
<210> 31
   <211> 9
   <212> PRT
   <213> Plasmodium falciparum
<400> 31
   Leu Ser Ser Gln Leu Leu Glu Glu Lys
   1 5
<210> 32
   <211> 12
   <212> PRT
   <213> Plasmodium falciparum
<400> 32
   Thr Val Tyr Asp Asp Gly Val Ile Tyr Asp Ala Lys
   1 5 10
<210> 33
   <211> 8
   <212> PRT
   <213> Plasmodium falciparum
<400> 33
   Asn Ile Leu Val Glu Leu Val Lys
   1 5
<210> 34
   <211> 10
   <212> PRT
   <213> Plasmodium falciparum
<400> 34
   Ala Ser Gln Ala Met Pro Thr Val Ile Lys
   1 5 10
<210> 35
   <211> 10
   <212> PRT
   <213> Plasmodium falciparum
<400> 35
   Asn Thr Pro Ser Ser Ser Gly Leu Val Lys
   1 5 10
<210> 36
   <211> 12
   <212> PRT
   <213> Plasmodium falciparum
<400> 36
   Asn Val Cys Thr Glu Ala Gly Met Phe Ala Ile Arg
   1 5 10
<210> 37
   <211> 9
   <212> PRT
   <213> Plasmodium falciparum
<400> 37
   Gln Leu Glu Gln Asp Ile Gln Leu Lys
   1 5
<210> 38
   <211> 9
   <212> PRT
   <213> Plasmodium falciparum
<400> 38
   Thr Cys Val Ser Gln Ala Leu Ser Lys
   1               5
<210> 39
   <211> 10
   <212> PRT
   <213> Plasmodium falciparum
<400> 39
   Ser Ser Leu Ser Ser Lys Pro Gly Val Lys
   1               5                   10
<210> 40
   <211> 7
   <212> PRT
   <213> Plasmodium falciparum
<400> 40
   Phe Leu Asn Ile Asp Ser Lys
   1               5
<210> 41
   <211> 10
   <212> PRT
   <213> Plasmodium falciparum
<400> 41
   Ser Thr Thr Thr Gly His Ile Ile Tyr Lys
   1               5                   10
<210> 42
   <211> 16
   <212> PRT
   <213> Plasmodium falciparum
<400> 42
   Asp Leu Val Asn Asp Ala Asn Val Asp Ala Asp Glu Ser Gly Leu Lys
   1               5                   10                  15
<210> 43
   <211> 9
   <212> PRT
   <213> Plasmodium falciparum
<400> 43
   Glu Ser Thr Leu His Leu Val Leu Arg
   1 5
<210> 44
   <211> 13
   <212> PRT
   <213> Plasmodium falciparum
<400> 44
   Ile Gln Asp Lys Glu Gly Ile Pro Pro Asp Gln Gln Arg
   1 5 10
<210> 45
   <211> 9
   <212> PRT
   <213> Plasmodium falciparum
<400> 45
   Thr Leu Ser Asp Tyr Asn Ile Gln Lys
   1 5
<210> 46
   <211> 12
   <212> PRT
   <213> Plasmodium falciparum
<400> 46
   Asn Leu Val Pro Gly Glu Ser Val Tyr Gly Glu Lys
   1 5 10
<210> 47
   <211> 8
   <212> PRT
   <213> Plasmodium falciparum
<400> 47
   Leu Thr Tyr Glu Gln Ile Glu Lys
   1 5
<210> 48
   <211> 13
   <212> PRT
   <213> Plasmodium falciparum
<400> 48
   Ala His Asn Glu Phe Ile Ser Asn Leu Pro Asn Ile Lys
   1 5 10
<210> 49
   <211> 20
   <212> PRT
   <213> Plasmodium falciparum
<400> 49
   Ile Val His Asp Asn Pro Asp Thr Asn Asp Glu Asp Ile Asn Met Gln
   1               5                   10                  15
   Asn Leu Phe Lys
               20
<210> 50
   <211> 15
   <212> PRT
   <213> Plasmodium falciparum
<400> 50
   Glu Glu Asn Ser Asn Tyr Pro His Glu Leu Leu Asn Thr Ile Lys
   1               5                   10                  15
<210> 51
   <211> 15
   <212> PRT
   <213> Plasmodium falciparum
<400> 51
   Val Leu Ser Gly Glu Phe Phe Val Val Asn Pro His Leu Leu Lys
   1               5                   10                  15
<210> 52
   <211> 12
   <212> PRT
   <213> Plasmodium falciparum
<400> 52
   Thr Ala Thr Pro Glu Gln Ala Gln Leu Val His Lys
   1               5                   10
<210> 53
   <211> 9
   <212> PRT
   <213> Plasmodium falciparum
<400> 53
   Asn Ile Ala Gln Pro Ile Ile Asn Arg
   1               5
<210> 54
   <211> 8
   <212> PRT
   <213> Plasmodium falciparum
<400> 54
   Val His Ile Glu Tyr Gly Glu Lys
   1               5
<210> 55
   <211> 7
   <212> PRT
   <213> Plasmodium falciparum
<400> 55
   His Tyr Leu Ile His Leu Arg
   1 5
<210> 56
   <211> 12
   <212> PRT
   <213> Plasmodium falciparum
<400> 56
   Ala Gln Phe Met Gly Asp Ser Glu Leu Ala Gly Arg
   1 5 10
<210> 57
   <211> 8
   <212> PRT
   <213> Plasmodium falciparum
<400> 57
   Lys Val Ser Val Phe Ala Glu Lys
   1 5
<210> 58
   <211> 10
   <212> PRT
   <213> Plasmodium falciparum
<400> 58
   Tyr Glu Glu Val Ala Leu Glu Ile Lys Lys
   1 5 10
<210> 59
   <211> 12
   <212> PRT
   <213> Plasmodium falciparum
<400> 59
   Gly Ile Leu Leu Ser Gly Glu Pro Gly Thr Gly Lys
   1 5 10
<210> 60
   <211> 10
   <212> PRT
   <213> Plasmodium falciparum
<400> 60
   Glu Ala Leu Gly Val Ala Leu Asp Ala Arg
   1 5 10
<210> 61
   <211> 9
   <212> PRT
   <213> Plasmodium falciparum
<400> 61
   Lys Gly Ile Val Asn Ile Glu Thr Lys
   1 5
<210> 62
   <211> 14
   <212> PRT
   <213> Plasmodium falciparum
<400> 62
   Ala Thr Ile Ala Gly Gly Gly Val Ile Pro His Ile His Lys
   1 5 10
<210> 63
   <211> 12
   <212> PRT
   <213> Plasmodium falciparum
<400> 63
   Thr Leu Ile Pro Gly Tyr Ala Ser Tyr Thr Asn Lys
   1 5 10
<210> 64
   <211> 9
   <212> PRT
   <213> Plasmodium falciparum
<400> 64
   Leu Gly Gln Ser Met Glu Ala Val Arg
   1 5
<210> 65
   <211> 13
   <212> PRT
   <213> Plasmodium falciparum
<400> 65
   Ala Gly Ser Cys Gly Ser Leu Gln Pro Asp Leu Ile Lys
   1 5 10
<210> 66
   <211> 13
   <212> PRT
   <213> Plasmodium falciparum
<400> 66
   Val Val Cys Asp Ser Tyr Val Asp Leu Ala Tyr Asn Arg
   1 5 10
<210> 67
   <211> 11
   <212> PRT
   <213> Plasmodium falciparum
<400> 67
   Ile Ile Leu Ile Gly Asp Ser Gly Val Gly Lys
   1 5 10
<210> 68
   <211> 9
   <212> PRT
   <213> Plasmodium falciparum
<400> 68
   Asn Asn Asn Thr His Asp Leu Ile Lys
   1 5
<210> 69
   <211> 13
   <212> PRT
   <213> Plasmodium falciparum
<400> 69
   Ile Asn Phe Leu Val Pro Gly Asn Gln Gly Val Val Lys
   1 5 10
<210> 70
   <211> 13
   <212> PRT
   <213> Plasmodium falciparum
<400> 70
   Ile Asn Phe Leu Val Pro Gly Asn Gln Gly Val Val Lys
   1 5 10
<210> 71
   <211> 10
   <212> PRT
   <213> Plasmodium falciparum
<400> 71
   Leu Val Ala Val Glu Gly Val Val Asn Lys
   1 5 10
<210> 72
   <211> 14
   <212> PRT
   <213> Plasmodium falciparum
<400> 72
   Glu Asp Ile Glu Ile Glu Ser Ser His Met Pro His Phe Lys
   1 5 10
<210> 73
   <211> 11
   <212> PRT
   <213> Plasmodium falciparum
<400> 73
   Val Asn Asn Asn Leu Leu Asn Ser Ile Glu Lys
   1 5 10
<210> 74
   <211> 12
   <212> PRT
   <213> Plasmodium falciparum
<400> 74
   Ile Asp Glu Ile Val Leu Val Gly Gly Ser Thr Arg
   1 5 10
<210> 75
   <211> 15
   <212> PRT
   <213> Plasmodium falciparum
<400> 75
   His Lys Tyr Asn Thr Asn Thr Asp Ser Asn Asn Ile Asp Ile Lys
   1 5 10 15
<210> 76
   <211> 8
   <212> PRT
   <213> Plasmodium falciparum
<400> 76
   Gln Ile Pro Tyr Thr Met Ala Lys
   1 5
<210> 77
   <211> 8
   <212> PRT
   <213> Plasmodium falciparum
<400> 77
   Met Ile Gly Glu Ala Trp Asn Lys
   1 5
<210> 78
   <211> 10
   <212> PRT
   <213> Plasmodium falciparum
<400> 78
   Ser Asn Phe Ile Gln Glu Ile His Lys Lys
   1 5 10
<210> 79
   <211> 8
   <212> PRT
   <213> Plasmodium falciparum
<400> 79
   Ile Tyr Leu Glu Asp Asn Ile Lys
   1 5
<210> 80
   <211> 8
   <212> PRT
   <213> Plasmodium falciparum
<400> 80
   Glu Val Asn Thr Pro Phe Phe Lys
   1 5
<210> 81
   <211> 9
   <212> PRT
   <213> Plasmodium falciparum
<400> 81
   Tyr Ser Ser Leu Asn Asp Ile Asp Lys
   1 5
<210> 82
   <211> 11
   <212> PRT
   <213> Plasmodium falciparum
<400> 82
   Ile Trp His His Thr Phe Tyr Asn Glu Leu Arg
   1 5 10
<210> 83
   <211> 11
   <212> PRT
   <213> Plasmodium falciparum
<400> 83
   Ile Trp His His Thr Phe Tyr Asn Glu Leu Arg
   1 5 10
<210> 84
   <211> 7
   <212> PRT
   <213> Plasmodium falciparum
<400> 84
   Arg Gly Ile Leu Thr Leu Lys
   1 5
<210> 85
   <211> 10
   <212> PRT
   <213> Plasmodium falciparum
<400> 85
   Gly His Ile Leu Glu Val Asp Ile Lys Lys
   1 5 10
<210> 86
   <211> 11
   <212> PRT
   <213> Plasmodium falciparum
<400> 86
   Ile Gly Gly Ile Gly Thr Val Pro Val Gly Arg
   1 5 10
<210> 87
   <211> 7
   <212> PRT
   <213> Plasmodium falciparum
<400> 87
   Thr Ile Glu Lys Phe Glu Lys
   1 5
<210> 88
   <211> 13
   <212> PRT
   <213> Plasmodium falciparum
<400> 88
   Gly Ile Leu Ala Ala Asp Glu Ser Thr Gln Thr Ile Lys
   1 5 10
<210> 89
   <211> 9
   <212> PRT
   <213> Plasmodium falciparum
<400> 89
   Val Leu Val Pro Asn Gly Val Ile Lys
   1 5
<210> 90
   <211> 13
   <212> PRT
   <213> Plasmodium falciparum
<400> 90
   Asp Leu Asp His Cys Cys Leu Val Asn Asp Glu Gly Lys
   1 5 10
<210> 91
   <211> 13
   <212> PRT
   <213> Plasmodium falciparum
<400> 91
   His Val Leu Ile Val Glu Asp Ile Ile Asp Thr Gly Lys
   1 5 10
<210> 92
   <211> 20
   <212> PRT
   <213> Plasmodium falciparum
<400> 92
   Ile His Asn Tyr Ser Ala Val Glu Thr Ser Lys Pro Leu Phe Gly Glu
   1 5 10 15
   His Tyr Val Arg
   20
<210> 93
   <211> 8
   <212> PRT
   <213> Plasmodium falciparum
<400> 93
   Gly Phe Phe Thr Ala Leu Leu Lys
   1 5
<210> 94
   <211> 7
   <212> PRT
   <213> Plasmodium falciparum
<400> 94
   Leu Ala Tyr Asp Ile Lys Lys
   1 5
<210> 95
   <211> 9
   <212> PRT
   <213> Plasmodium falciparum
<400> 95
   Val Leu Val Pro Asn Gly Val Ile Lys
   1 5
<210> 96
   <211> 10
   <212> PRT
   <213> Plasmodium falciparum
<400> 96
   His Tyr Gly Ser Leu Gln Gly Leu Asn Lys
   1 5 10
<210> 97
   <211> 12
   <212> PRT
   <213> Plasmodium falciparum
<400> 97
   Phe Thr Gly Trp Thr Asp Val Pro Leu Ser Glu Lys
   1 5 10
<210> 98
   <211> 11
   <212> PRT
   <213> Plasmodium falciparum
<400> 98
   Thr Ala Asp Leu Leu His Val Pro Val Val Lys
   1 5 10
<210> 99
   <211> 11
   <212> PRT
   <213> Plasmodium falciparum
<400> 99
   His Gly Glu Ser Thr Trp Asn Lys Glu Asn Lys
   1 5 10
<210> 100
   <211> 18
   <212> PRT
   <213> Plasmodium falciparum
<400> 100
   Val Leu Pro Phe Trp Phe Asp His Ile Ala Pro Asp Ile Leu Ala Asn
   1               5                   10                  15
   Lys Lys
<210> 101
   <211> 10
   <212> PRT
   <213> Plasmodium falciparum
<400> 101
   Ala Ile Cys Thr Ala Trp Asn Val Leu Lys
   1               5                   10
<210> 102
   <211> 8
   <212> PRT
   <213> Plasmodium falciparum
<400> 102
   Lys Tyr Gly Glu Glu Gln Val Lys
   1               5

## Description

The present invention relates to the biomarker PF10_0121 for determining the malarial status of a subject, and to uses thereof. The present invention is defined by the appended claims. Any subject-matter identified in the application as "invention", "embodiment", "aspect", etc., which exceeds the scope of the invention as represented by the claims does not form part of the claimed invention but only serves as background information to better understand the invention.

Human malaria is a life-threatening disease caused by Plasmodium parasites which accounts for approximately 655,000 deaths yearly, primarily in African children. Five Plasmodium species cause human malaria, but the majority of deaths are attributable to *Plasmodium falciparum.* Since most deaths in children admitted to hospital with severe malaria occur within the first 24 hours, it is critical to diagnose and treat malaria patients promptly.

The standard method to diagnose malaria infection requires the visualization of the parasite in blood using light microscopy. However, laboratory facilities to diagnose malaria are not available in many resource-poor endemic settings, and the administration of antimalarials is often based on non-specific clinical symptoms. Treatment allocation based on clinical presentation results in over diagnosis of malaria and thus in the overuse of antimalarial treatment, which contributes to the increasing resistance of *Plasmodium falciparum* to available drugs. Consequently, the World Health Organisation recommends that all suspected malaria cases should be confirmed with a parasite-based diagnostic assay. A number of commercially available rapid diagnostic tests (RDTs) based on lateral flow immunochromatographic assays (dipsticks) that detect *Plasmodium falciparum* proteins have been developed and implemented with variable success. RDTs are relatively inexpensive, simple to use and results can be obtained in approximately 20 minutes.

The most widely available RDTs used in malaria detect histidine-rich protein 2 (HRP-2), lactate dehydrogenase (pLDH) and aldolase (pFBPA). Whilst these markers have value they do have limitations. For example, the HRP2 antigen may persist in blood after treatment with antimalarials for up to 33 days, which invalidates the use of this test to monitor treatment success. Similarly, the identification of HRP-2 in current and recent infections limits the diagnostic value of HRP-2 in malaria-endemic areas with high transmission intensity. Moreover, parasite populations with deletions in the histidine rich repeat region of the *hrp2* gene have been documented. pLDH does not persist in blood but *Plasmodium falciparum* gametocytes, the sexual stage of malaria parasites that does not cause acute infection, produce pLDH and this may produce a positive test despite clearance of the asexual parasite forms that have caused the acute infection.

Huang et al. 2012, discloses proteomic identification of host and parasite biomarkers in saliva with uncomplicated malaria.

There therefore remains a need for additional biomarkers for malaria.

It is an aim of the present invention therefore to provide one or more biomarker(s) that may be used to give an indication of the malarial status in an individual and/or which may also be used to assess the type of treatment that is appropriate for such an individual.

In a first aspect, the present invention provides a method for determining the malarial status of a subject, comprising the steps of:
i. providing a biological sample obtained from a subject;
ii. determining the level, or presence, of PF10_0121 (hypoxanthine phosphoribosyltransferase, pHPRT) and/or PF11_0208 (phosphoglycerate mutase, pPGM) in the biological sample.

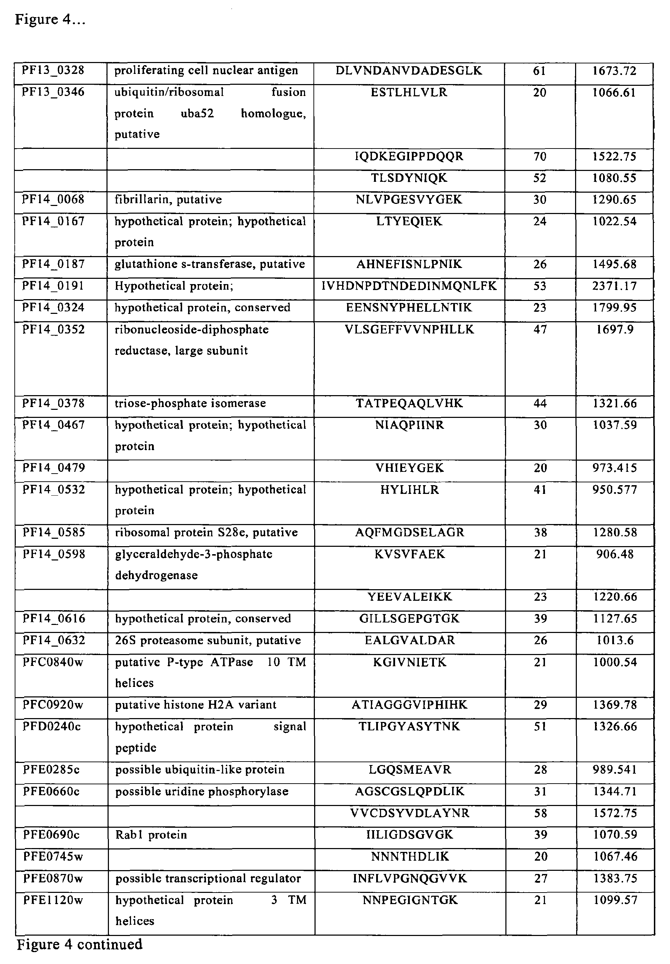

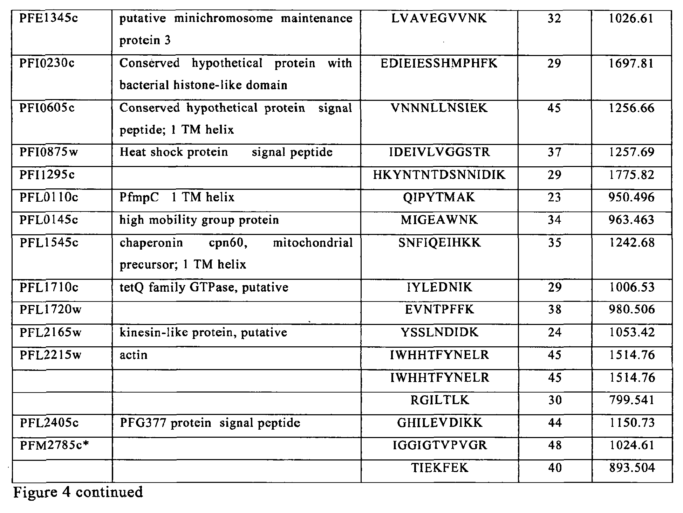

In an alternative embodiment, in step (ii) of the method of the invention the level, or presence, of one or more of the proteins identified in Figure 4 is determined. The proteins identified in Figure 4 may be defined by the name given in the table and/or by the accession number given in the table, the proteins are referred to herein as the "alternative biomarkers". The alternative biomarker may be any protein identified in Figure 4, in particular it may be any one or more of the following: 14-3-3 protein, heat shock protein 86, heat shock 70kDa protein, QF122 antigen, enolase, ribosomal phosphoprotein P0, vacuolar ATP synthase catalytic subunit a, elongation factor 1 alpha, proliferating cell nuclear antigen, ribonucleoside-diphosphate reductase large subunit, triose-phosphate isomerise, glyceraldehyde-3-phosphate dehydrogenase, Rab1, heat shock protein signal peptide, PfmpC 1 TM helix, high mobility group protein, chaperonin cpn60 mitochondrial precursor and actin.

Preferably the presence of pHPRT and/or pPGM and/or any of the alternative biomarkers is indicative/diagnostic of malaria in a subject.

The method of the invention may include a further step of concluding a subject has malaria if pHPRT and/or pPGM and/or one or more of the alternative biomarkers are present in the sample. As both pHPRT and pPGM and the alternative biomarkers are parasite proteins they should not be present at any level in a subject who does not have malaria.

The method of the invention may further comprise the step of comparing the level determined in (ii) with one or more pre-determined reference values.

The level of pHPRT and/or pPGM and/or one or more of the alternative biomarkers may be compared to the level in a control sample, preferably a non-infected sample, to allow for any inaccuracy or background in the test method used.

In a preferred embodiment in step (ii) the level of at least pHPRT is determined

The method of the invention may allow the diagnosis of acute or symptomatic malaria, that is, the presence of pHPRT and optionally also pPGM and/or one or more of the alternative biomarkers, may allow a diagnosis of acute malaria. Wherein acute malaria is defined as the presence of malarial parasites and symptoms of malaria, for example a fever.

The step of obtaining the sample preferably does not form part of the invention

The term 'biological sample' refers to a sample of biological fluid obtained for the purpose of diagnosis or evaluation of a subject of interest. Preferred biological samples include, but are not limited to, blood, serum, plasma, saliva and cerebrospinal fluid. In addition, the person skilled in the art would realise that some test samples would be more readily analyzed following a fractionation or purification procedure, for example, separation of whole blood into serum or plasma components.

The biological sample may be a plasma sample obtained from a subject

The term 'level' as defined herein refers to the amount or concentration of pHPRT and/or pPGM and/or one or more of the alternative biomarkers contained in the biological sample.

The phrase "malarial status" includes any manifestation of malaria. For example, the presence or absence of malaria, the progression of malaria, and the effectiveness or response of a subject to a treatment for malaria.

The method of the invention may be used, for example, for any one or more of the following: to diagnose malaria; to advise on the prognosis of a subject with malaria; to monitor disease progression; and to monitor effectiveness or response of a subject to a particular treatment.

Preferably the method of the invention allows the diagnosis of malaria from the analysis of the level of pHPRT and/or pPGM and/or one or more of the alternative biomarkers in a sample from the patient. Preferably at least pHPRT levels are measured.

The level of pHPRT and/or pPGM and/or one or more of the alternative biomarkers may be compared to a reference value. Wherein the reference value be the level of expression of the same protein in a sample from one or more subjects who do not have malaria - as determined, for example, by the visualisation of the parasite light microscopy. These samples have so called "normal values" of the biomarkers.

Alternatively, the reference value may be a previous value obtained for a specific subject. This kind of reference may be used if the method is to be used to monitor progression of an infection or to monitor response of a subject to a particular treatment.

The level of pHPRT and/or pPGM and/or one or more of the alternative biomarkers may be evaluated by any suitable method. For example if protein levels are to be determined any of the group comprising immunoassays, spectrometry, western blot, ELISA, immunoprecipitation, slot or dot blot assay, isoelectric focussing, SDS-PAGE and antibody microarray immunohistological staining, radio immuno assay (RIA), fluoroimmunoassay, an immunoassay using an avidin-biotin or streptoavidin-biotin system, etc and combinations thereof may be used. These methods are well known to persons skilled in the art. Other methods may also be used.

It may be sufficient to simply determine whether pHPRT and/or pPGM and/or one or more of the alternative biomarkers are present in a sample, absolute values may not be necessary.

Preferably the method of the invention allows a result to be obtained in less than 24 hours, preferably less than 12 hours, less 6 hours, less than 4 hours, less than 3 hours, less than 2 hours, less than 1 hour, more preferably less than 30 minutes.

The method of the invention may be used in conjunction with an assessment of clinical symptoms to provide a more effective diagnosis of malaria.

The present invention may also provide a method for determining the appropriate treatment for a subject comprising the steps of:
(a) providing a biological sample obtained from a subject;
(b) determining the level, or presence, of pHPRT and/or pPGM in the biological sample from said subject; and
(c) using the results in (b) to determine the most appropriate therapy.

In an alternative embodiment, in step (b) the level, or presence, of one or more of the alternative biomarkers may be determined.

For example, a patient with pHPRT and/or pPGM and/or one or more of the alternative biomarkers in the sample may be treated as having malaria and anti-malarial drugs will be administered.

The method of the invention may include a further step of comparing the level of pHPRT and/or pPGM and/or one or more of the alternative biomarkers determined in step (b) with one or more predetermined reference values prior to step (c). Preferably the level of pHPRT is determined.

In another embodiment of the invention, the method of the invention which uses pHPRT and/or pPGM and/or one or more of the alternative biomarkers to determine a diagnosis of malaria may be used in combination with the clinical features, for example fever, to determine a diagnosis of malaria.

The method of the present invention may be carried out *in vitro.*

The subject may be a human. In another embodiment the subject may be a mammal, for example, a dog, cat, horse, cow, monkey, ape, rodent, hamster, rat, or guinea pig.

According to another aspect of the invention, the invention provides a method of determining the response of a subject with malaria to a particular treatment comprising the steps of:
(a) providing a biological sample obtained from a subject;
(b) determining the level of pHPRT and/or pPGM in the biological sample from said subject; and
(c) comparing the level of pHPRT and/or pPGM determined in step (b) with one or more reference values.

In an alternative embodiment, in step (b) the level of one or more of the alternative biomarkers may be determined, and in step (c) the level of one or more of the alternative biomarkers is compared with a reference value.

The reference value may be the level of pHPRT and/or pPGM and/or one or more of the alternative biomarkers in an earlier sample from the same subject.

According to another aspect of the invention, the invention provides a method for treating malaria in a patient comprising: requesting a test providing the results of an analysis to determine whether the patient has pHPRT and/or pPGM in a biological sample from the patient and administering antimalarial treatment to the patient if pHRT and/or pPGM are found in the biological sample.

According to a still further aspect, the invention provides a method of diagnosing malaria in a human subject, wherein the malaria is characterised by the presence of pHRT and/or pPGM comprising:
i) obtaining a biological sample from the subject;
ii) applying a monoclonal antibody specific for pHRT or pPGM, wherein the presence of pHPRT or pPGM creates an antibody-biomarker complex;
iii) applying a detection agent that detects the antibody-biomarker complex; and
iv) diagnosing malaria where the detection agent in step (iii) is detected.

According to yet another aspect of the invention, the invention provides a kit for use in determining the malarial status of a subject comprising at least one agent for determining the level of pHPRT and/or pPGM and/or one or more of the alternative biomarkers in a biological sample provided by the subject.

The agent may be an antibody

The kit may further comprise instructions for suitable operational parameters in the form of a label or separate insert.

The kit may further comprise one or more pHPRT and/or pPGM samples to be used as standard(s) for calibration and comparison.

The invention may further provide use of the level of pHPRT and/or pPGM and/or one or more of the alternative biomarkers as a biomarker to determine the malarial status of a subject.

According to another aspect the invention may provide a method of diagnosing malaria in a subject comprising:
i. obtaining a biological sample obtained from a subject;
ii. determining the presence, and optionally the level, of PF10₋0121 (hypoxanthine phosphoribosyltransferase, pHPRT) and/or PF11_0208 phosphoglycerate mutase, pPGM) in the biological sample; and
iii. diagnosing malaria if pHPRT and/or pPGM are present in the sample.

In an alternative embodiment, in step (ii) the presence, and optionally the level, of one or more the alternative biomarkers is determined, and this is used in step (iii) to diagnose malaria.

Preferably at least the level of pHPRT is determined

According to another aspect the invention may provide a method of treating malaria in a subject comprising:
i. obtaining a biological sample obtained from a subject;
ii. determining the presence, and optionally the level, of PF10_0121 (hypoxanthine phosphoribosyltransferase, pHPRT) and/or PF11_0208 phosphoglycerate mutase, pPGM) in the biological sample; and
iii. administering anti-malarial drugs if pHPRT and/or pPGM are present in the sample.

In an alternative embodiment, in step (ii) the presence, and optionally the level, of one or more the alternative biomarkers is determined, and this is used in step (iii) to determine if anti-malarial drugs should be administered.

The skilled man will appreciate that preferred features of any one embodiment and/or aspect of the invention may be applied to all other embodiments and/or aspects of the invention.

There now follows by way of example only a detailed description of the present invention with reference to the accompanying drawings, in which:

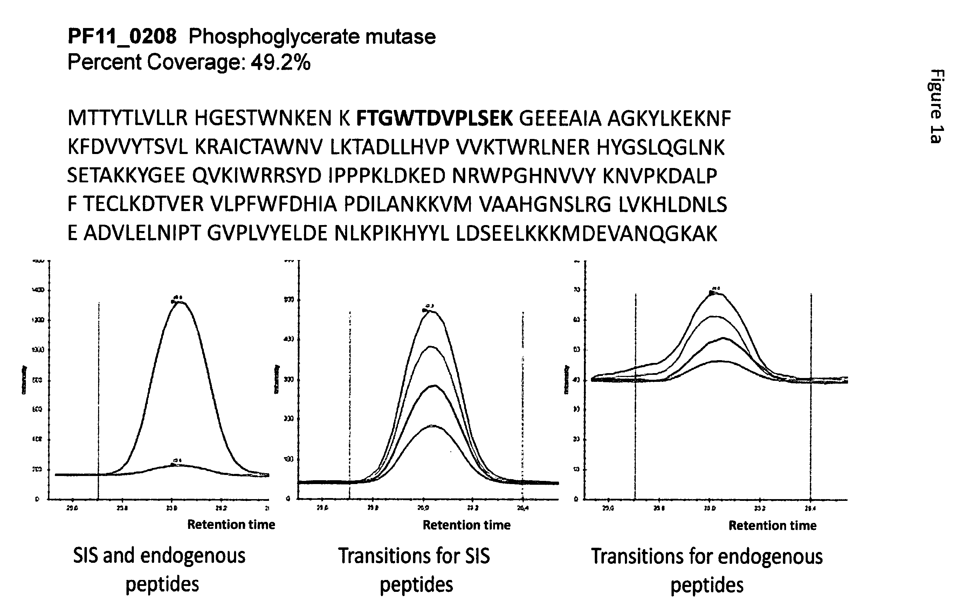

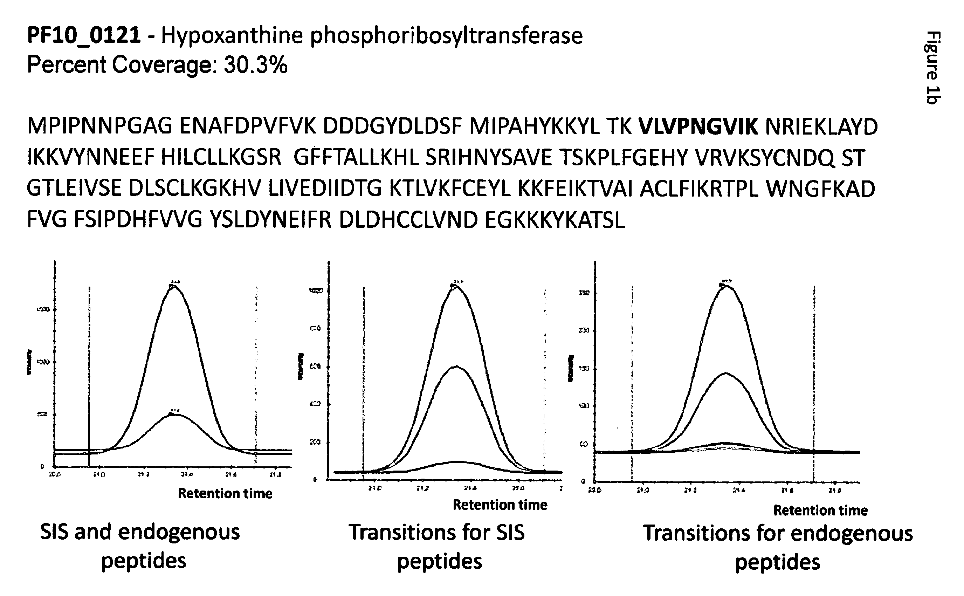

**Figure 1** **-** details the *Plasmodium falciparum* proteins pPGM (Figure 1a - Seq ID No: 1) and pHPRT (Figure 1b - Seq ID No: 2). In each figure the top panel shows protein sequence coverage and peptide sequences used to identify parasite proteins with high confidence (highlighted and underlined). The lower panel shows a representative optimised SRM assay for selected proteotypic peptides. The left panel shows the relative abundance of heavy-labelled stable isotopic peptides (SIS) versus endogenous peptides in plasma. The middle panel shows intensity of the transitions measured for SIS peptides in plasma and the right panel shows the intensity of endogenous transitions measured in the plasma of a representative individual.

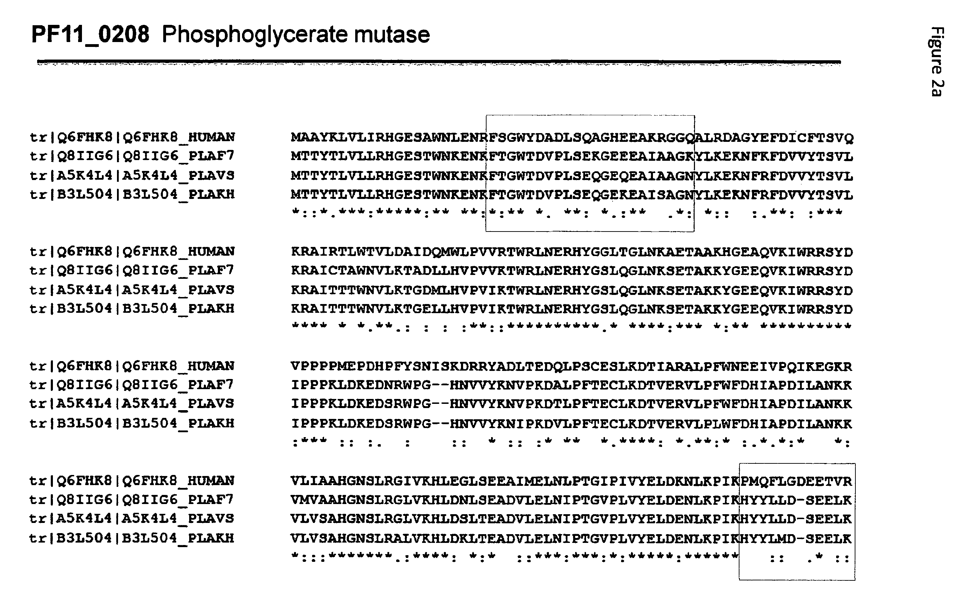

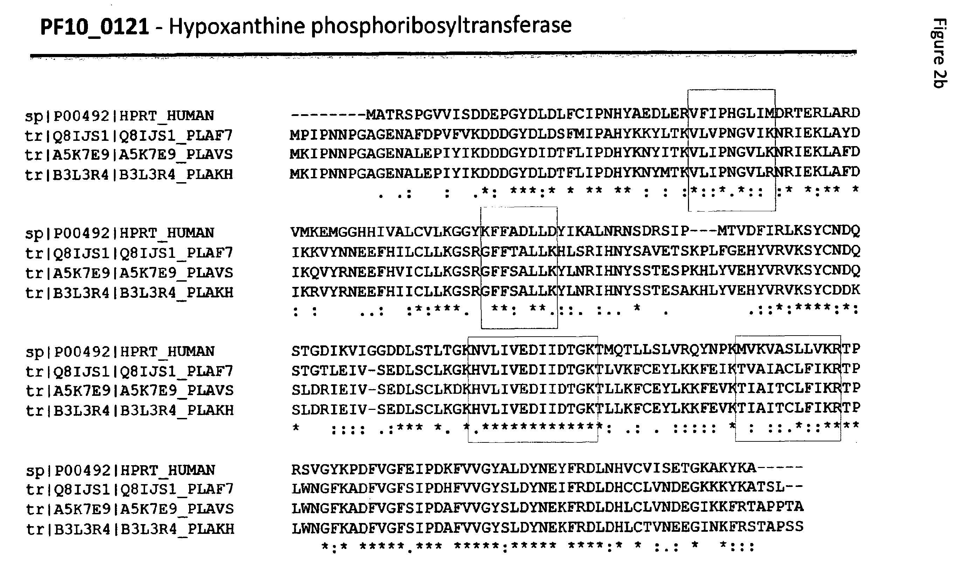

**Figure 2** - shows a sequence alignment of *Plasmodium falciparum* proteins pPGM (Figure 2a - Seq ID Nos: 3, 4, 5 and 6) and pHPRT (Figure 2b - Seq ID No: 7, 8, 9 and 10) and selected proteotypic peptides and corresponding protein sequences in *Homo sapiens.* Data show protein alignment for parasites that may cause human malaria (*P. falciparum, P. vivax, P.ovale, P. malariae* and *P. knowlesi*) and *Homo sapiens.*

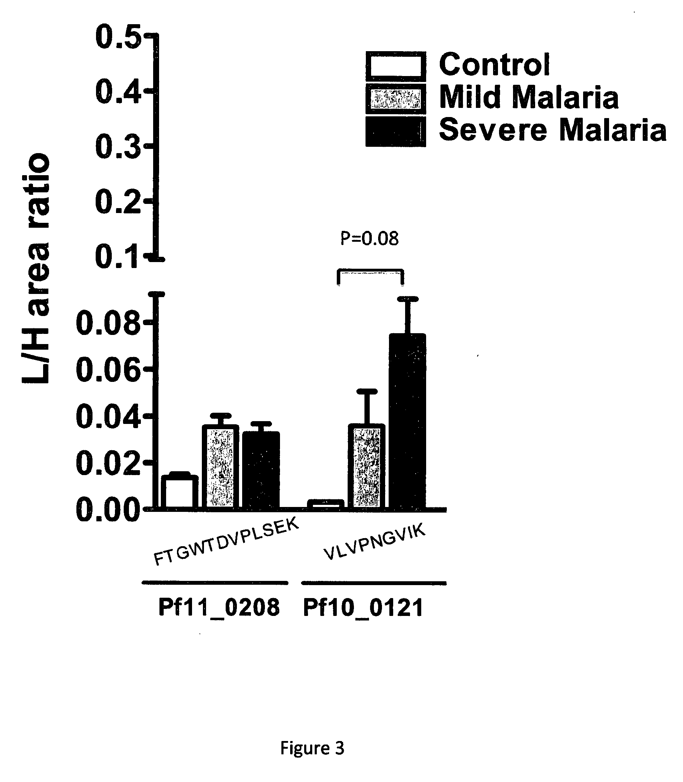

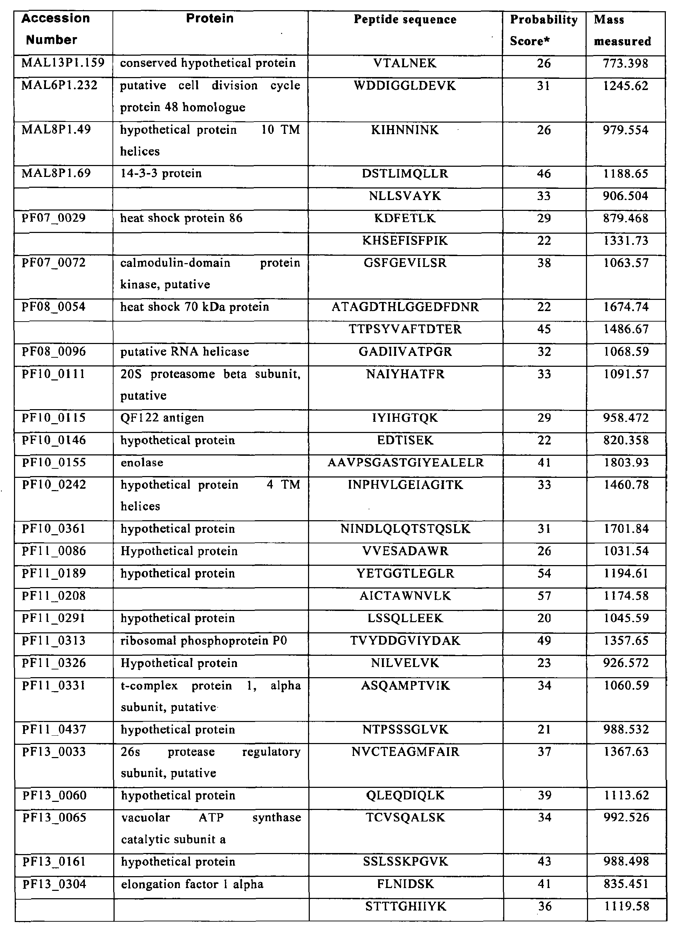

**Figure 3** **-** illustrates the quantification of *Plasmodium falciparum* proteotypic peptides (PTPs) using SRM assays. Data show the mean ratio of light (endogenous) to heavy-labelled peptides (L/H area ratio) corresponding to the *Plasmodium falciparum* proteins pPGM and pHPRT measured in plasma samples from Gambian children with severe malaria (N=15), mild malaria (N=15) and convalescent controls (28 days after treatment) (N=15). Error bars indicate standard error of the mean. *** P<0.001, and NS: non-significant.
**Figure** 4 - is a table detailing *P. falciparum* proteins identified in plasma samples from children with severe malaria. * A score of >20 is acceptable for protein identification. The peptide sequences referred to are included in the sequence listing as Seq ID Nos: 11-87)

### Methods

### Samples

Sample processing and storage were performed according to standard operating procedures at the MRC Laboratories in the Gambia which follow good clinical and laboratory practice (GCLP) regulations. Blood samples were processed within 2 hours of collection. Plasma was separated and stored at -80°C.

### Selective reaction monitoring (SRM) validation assays

*Sample preparation.* Twelve microlitres of plasma samples from 48 individual patients were delipidated and depleted from the 14 most abundant proteins (MARS Human-14, Agilent Technologies), precipitated and quantified.

*Peptide selection and synthesis.* For each protein three to five proteotypic peptides (PTP) were selected. Based on the y-series fragment ions, three to five transitions were included for each of the two main charge states for each peptide. The predicted PTPs were synthesized (unpurified) with isotopically-labeled amino acids either with a C-term Arg (¹³C₆ ¹⁵N₄) or a C-term Lys (¹³C₆ ¹⁵N₂) using SPOT synthesis (JPT Peptide Technology, Germany) and used as a reference to develop the corresponding SRM assays. The mass difference between the endogenous and the heavy labeled peptides for the 1+ fragment was either 8 or 10 Da. The stable isotope standard peptides were added prior to protein digestion and used as internal standards.

Purified heavy labelled peptides were synthesized on an APEX396 solid phase synthesizer (AAPPTec, Louisville, KY, US). A 9-fluorenylmethyloxycarbonyl (Fmoc) strategy was used in the synthesis of the peptides. Initially, the first Fmoc amino acid was attached to an insoluble support resin via an acid labile linker. Deprotection of the Fmoc group was accomplished by treatment of the amino acid with 20% piperidine in dimethylformamide (DMF). The second Fmoc amino acid was coupled utilizing in situ activation of the amino acid to the growing peptide chain. After the desired peptide is synthesized, the resin bound peptide was deprotected and detached from the solid support via trifluoroacetic acid (TFA) cleavage. Peptides were precipitated with ether, dried and analysed on a reversed phase HPLC column and MALDI-TOF mass spectrometer (Ultraflex, Bruker Daltonics, Germany).

*Peptide SRM standard curve and linear range.* The peptides were weighed with high precision scale, solubilized in 10% ACN and 0.5% FA and dried in a speed vacuum centrifuge. Serial dilutions from a stock concentration of 23 ng/µL were produced for the peptides GILAADESTQTIK (Seq ID No: 88) and VLVPNGVIK (Seq ID No: 89) in 2% ACN and 0.1% FA.

*SRM* assays: For SRM assays, 2 to 3 peptides and 3 to 5 transitions per peptide were used to target the pPGM or pHPRT proteins. Depleted plasma samples were spiked with a mixture of stable isotope standard (SIS) peptides in equimolar amount to get 109.1 fmol of each SIS peptide and 1 µg of plasma proteins in 1 µL. The mixture was reduced with 200 mM DTT in 100 mM Tris buffer, pH 7.8 for 30 min, alkylated with 200 mM iodoacetamide in 100 mM Tris buffer, pH 7.8 for 30 min followed by second 30 minutes incubation with DTT. The proteins were digested with sequencing grade modified trypsin (Promega) at 37°C overnight using an enzyme to substrate ratio of 1:20. The digestion was stopped by adding 5% formic acid. Samples were desalted by Sep-Pak Light C18 cartridge (Waters), dried by speed vacuum and resuspended in buffer A (2% ACN, 0.1% FA) to get a final concentration of 1 µg/µL for plasma sample and 109.1 fmol/µL for each heavy-labelled peptide. One µL was injected on the Agilent 6460 Triple Quadrupole instrument coupled to an Agilent 1200 HPLC system equipped with a Chip-cube (Agilent Technologies).

Two separate MRM methods with 45 or 47 transitions each were used to obtain cycle times at 1.8s and dwell-times at 35ms. The full width at half height was 0.3 min. The optimal collision energy (CE) used for SRM assays was derived from empirical data of SIS peptide fragmentation in a 6520 Q-TOF instrument (Agilent Technologies). Q-TOF MS/MS data from SIS peptides were searched against a FASTA file that included (1) a concatenated sequence of all synthetic peptides, (2) *P. falciparum* 3D7 and (3) *Homo sapiens* proteomes to search MS/MS data.

*HPLC*: Samples were analyzed on an Agilent 1200 Series HPLC-Chip (C18 chip withl60 nL trap column) using a 0.3 µl/min flow rate and a 60 minutes gradient (as for the shotgun experiment). Briefly, we used a linear gradient from 3 to 40% buffer B (95% ACN, 0.1% FA) for 46.67 min, followed by a sharp increase to 100% within 4 min, kept for 5.33 minutes followed by a linear gradient from 100 to 3 % for 4 min to finally equilibrate the system for 9 min with 3 % buffer B.

### Data analysis

SRM data were analyzed using Skyline software (v1.2) (MacLean, B., et al (2010) Skyline: an open source document editor for creating and analyzing targeted proteomics experiments. Bioinformatics 26, 966-968). Relative quantitation was done using the area ratio (light/heavy) and for each peptide only the 3 most intense transitions were considered. For each selected peptides, the most intense peak was used as quantifier and the others were used as qualifiers to validate the ratio and the retention time of the peptide measured. Retention times and peak selection was initially performed automatically by the software and validated by inspection of individual chromatograms, and selection was based on the retention time obtained with the heavy labelled peptides. The result was an alignment of the endogenous with the heavy labelled peptides. In those cases where transitions for endogenous peptides were not well resolved (eg, retention time mismatches), the analysis was discontinued.

Protein sequence alignment was performed using Clustal Omega and visualized in ClustalX v2.1. (Larkin, M. A et al (2007) Clustal W and Clustal X version 2.0. Bioinformatics 23, 2947-2948; Sievers, F., et al (2011) Fast, scalable generation of high-quality protein multiple sequence alignments using Clustal Omega. Mol Syst Biol 7, 539). The statistical analysis was done with GraphPad Prism 5 (GraphPad Software Inc, USA) and Stata 11 (StataCorp LP, USA).

### Results

### Selected Reaction Monitoring Assays to quantify P. falciparum proteins

To validate and quantify the *P. falciparum* proteins pPGM and pHPRT SRM assays were used. Initially 6 proteotypic peptides were selected for pHPRT and 8 were selected for pPGM (see Table 1). SRM-based quantification was performed only for those cases where both the heavy-labeled and the endogenous peptide consistently showed identical retention times and at least 3 transitions (n=45). These criteria were met by 1 proteotypic peptide for pPGM and pHPRT.

**Table 1**

| **Description** | **Peptides sequence** |
|---|---|
| PF10_0121 hypoxanthine phosphoribosyltransferase (HPRT) | DLDHCCLVNDEGK - Seq ID No: 90 |
| | HVLIVEDIIDTGK - Seq ID No: 91 |
| | IHNYSAVETSKPLFGEHYVR - Seq ID No: 92 |
| | GFFTALLK - Seq ID No: 93 |
| | LAYDIKK - Seq ID No: 94 |
| | **VLVPNGVIK** - Seq ID No: 95 |
| PF11 0208 phosphoglycerate mutase (PGM) | HYGSLQGLNK - Seq ID No: 96 |
| | **FTGWTDVPLSEK -** Seq ID No: 97 |
| | TADLLHVPVVK - Seq ID No: 98 |
| | HGESTWNKENK - Seq ID No: 99 |
| | VLPFWFDHIAPDILANKK - Seq ID No: 100 |
| | AICTAWNVLK - Seq ID No: 101 |
| | KYGEEQVK - Seq ID No: 102 |

The transitions detected for each peptide are shown in Figure 1. The SIS and the endogenous peptides were eluted at the same retention time. In the majority of cases, the intensity of the precursor was higher for the SIS peptide. However, despite lower intensity the number of transitions detected with endogenous peptides was higher than those of spiked peptides. Peptides were detected for all the proteins and in most cases analysed. The area of the transition with the highest intensity (quantifier) of the endogenous peptide (light) and the corresponding area of the heavy-labeled peptide (spiked) were used to calculate the ratios to perform relative quantitation of the peptides in individual samples. pPGM was measured with FTGWTDVPLSEK - Seq ID No: 96 peptide and could be quantified in 36 patients, and pHPRT was measured with VLVPNGVIK - Seq ID No: 94 and could be quantified in 28 patients.

The uniqueness criterion used for PTP selection of *P.falciparum* proteins was based on differences with host protein sequence (*Homo sapiens*) but not with other parasite species that may cause human malaria. The sequence alignment indicates that peptides selected to quantify *P. falciparum* PGM and HPRT were species specific.

### P. falciparum protein validation in plasma and clinical correlates

Using SRM, the parasite peptides were detected mostly in plasma from severe malaria cases (N=15) but were also found in plasma samples from mild malaria (N=15) and convalescent cases (N=15). The SRM assays for pHPRT indicated a significantly higher concentration of this protein in severe malaria cases as compared to mild malaria cases. pPGM was higher in acute malaria cases compared with convalescent controls (Figure 3).

## Claims

1. A method for determining the malarial status of a subject, comprising the steps of:
i) providing a biological sample obtained from a subject; and
ii) determining the level, or presence, of PF10_0121 (hypoxanthine phosphoribosyltransferase, pHPRT) in the biological sample.

2. The method of claim 1 wherein the presence of pHPRT is indicative/diagnostic of malaria in a subject.

3. The method of one or more preceding claim further comprising the step of comparing the level determined in step (ii) with one or more pre-determined reference values.

4. The method of one or more preceding claim wherein the presence of pHPRT is diagnostic of acute or symptomatic malaria.

5. The method of one or more preceding claim wherein the method is used for any one or more of the following: to diagnose malaria; to advise on the prognosis of a subject with malaria; to monitor disease progression; and to monitor effectiveness or response of a subject to a particular treatment.

6. A method for determining the appropriate treatment for a subject comprising the steps of:
(a) providing a biological sample obtained from a subject;
(b) determining the level, or presence, of pHPRT in the biological sample from said subject; and
(c) using the results in (b) to determine the most appropriate therapy

7. The method of claim 6 wherein the presence of pHPRT in the sample indicates that the subject should be treated as having malaria and anti-malarial drugs should be administered.

8. The method of claim 6 or 7 comprising a further step of comparing the level of pHPRT determined in step (b) with one or more predetermined reference values prior to step (c).

9. A method of determining the response of a subject with malaria to a particular treatment comprising the steps of:
(a) providing a biological sample obtained from a subject;
(b) determining the level of pHPRT in the biological sample from said subject; and
(c) comparing the level of pHPRT determined in step (b) with one or more reference values.

10. The method of claim 9 wherein the reference value is the level of pHPRT in an earlier sample from the same subject.

11. A method of diagnosing malaria in a subject comprising:
i) obtaining a biological sample obtained from a subject;
ii) determining the presence, and optionally the level, of PF10_0121 (hypoxanthine phosphoribosyltransferase, pHPRT) in the biological sample; and
iii) diagnosing malaria if pHPRT are present in the sample

12. An anti-malarial drug for use in treating malaria in a subject, wherein the anti-malarial drug is for administration when pHPRT is present in a sample obtained from the subject.

13. The method or anti-malarial drug of one or more preceding claim wherein the biological sample is a plasma sample.

## Patentansprüche

1. Ein Verfahren für die Bestimmung des Malaria-Status eines Patienten, das die folgenden Schritte aufweist:
i) die Bereitstellung einer biologischen Probe von einem Patienten; und
ii) die Bestimmung des Grades oder des Vorhandenseins von PF10_0121 (Hypoxanthin-Phosphoribosyltransferase, pHPRT) in der biologischen Probe.

2. Das Verfahren gemäß Anspruch 1, wobei das Vorhandensein von pHPRT Malaria in einem Patienten anzeigt oder diagnostiziert.

3. Das Verfahren gemäß eines oder mehrerer der vorhergehenden Ansprüche, die darüberhinaus den Schritt des Vergleichs des in Schritt (ii) mit einem oder mehreren vorher festgelegten Referenzwerten bestimmten Grades aufweist.

4. Das Verfahren eines oder mehrerer der vorhergehenden Ansprüche, wobei das Vorhandensein von pHPRT eine akute oder symptomatische Malaria anzeigt.

5. Das Verfahren gemäß eines oder mehrerer der vorhergehenden Ansprüche, wobei das Verfahren für eine oder mehrere der folgenden Aufgaben eingesetzt wird: für die Diagnose von Malaria; für die Beratung bei der Prognose eines Patienten mit Malaria; für die Überwachung des Krankheitsverlaufes; und für die Überwachung der Effektivität oder des Ansprechverhaltens eines Patienten auf eine bestimmte Behandlung.

6. Ein Verfahren für die Bestimmung der geeigneten Behandlung für einen Patienten, das die folgenden Schritte aufweist:
(a) die Bereitstellung einer biologischen Probe von einem Patienten;
(b) die Bestimmung des Grades oder des Vorhandenseins von pHPRT in der biologischen Probe; und
(c) die Verwendung der Resultate in (b) für die Bestimmung der geeignetsten Therapie.

7. Das Verfahren gemäß Anspruch 6, wobei das Vorhandensein von pHPRT in der Probe anzeigt, dass der Patient als Malaria-Patient behandelt werden muss und ihm Malaria-Medikamente verabreicht werden müssen.

8. Das Verfahren gemäß Anspruch 6 oder 7, das darüberhinaus den Schritt des Vergleichs des Grades an pHPRT, der in Schritt (b) mit einem oder mehreren vorher festgelegten Referenzwerten vor Schritt (c) bestimmt wurde, aufweist.

9. Ein Verfahren für die Bestimmung des Ansprechverhaltens eines Patienten mit Malaria auf eine bestimmte Behandlung, das die folgenden Schritte aufweist:
(a) die Bereitstellung einer biologischen Probe von einem Patienten;
(b) die Bestimmung des Grades oder des Vorhandenseins von pHPRT in der biologischen Probe des Patienten; und
(c) den Vergleich des Grades an pHPRT, der in Schritt (b) mit einem oder mehreren Referenzwerten bestimmt wurde.

10. Das Verfahren gemäß Anspruch 9, wobei der Referenzwert der Grad an pHPRT in einer früheren Probe desselben Patienten ist.

11. Ein Verfahren für die Diagnose von Malaria in einem Patienten, das Folgendes aufweist:
i) die Erlangung einer biologischen Probe von einem Patienten;
ii) die Bestimmung des Vorhandenseins, und optional des Grades an, PF10_0121 (Hypoxanthin-Phosphoribosyltransferase, pHPRT) in der biologischen Probe; und
iii) die Diagnose von Malaria, wenn pHPRT in der Probe vorhanden ist

12. Ein Malaria-Medikament für die Verwendung bei der Behandlung von Malaria in einem Patienten, wobei das Malaria-Medikament für die Verabreichung vorgesehen ist, wenn pHPRT in einer von einem Patienten erlangten Probe vorhanden ist.

13. Das Verfahren oder das Malaria-Medikament gemäß eines oder mehrerer der vorhergehenden Ansprüche, wobei die biologische Probe eine Plasma-Probe ist.

## Revendications

1. Un procédé de détermination de l'état paludéen d'un sujet, comprenant les opérations suivantes :
i) la fourniture d'un échantillon biologique obtenu auprès d'un sujet, et
ii) la détermination du niveau, ou de la présence, de PF10_0121 (hypoxanthine phosphoribosyltransférase, pHPRT) dans l'échantillon biologique.

2. Le procédé selon la Revendication 1 où la présence de pHPRT est indicative/un diagnostic de paludisme chez un sujet.

3. Le procédé selon l'une quelconque ou plusieurs des Revendications précédentes comprenant en outre l'opération de comparaison du niveau déterminé à l'opération (ii) à une ou plusieurs valeurs de référence prédéterminées.

4. Le procédé selon l'une quelconque ou plusieurs des Revendications précédentes où la présence de pHPRT est un diagnostic de paludisme aigu ou symptomatique.

5. Le procédé selon l'une quelconque ou plusieurs des Revendications précédentes où le procédé est utilisé pour l'une quelconque ou plusieurs des opérations suivantes :
diagnostiquer le paludisme, conseiller à propos du pronostic d'un sujet atteint de paludisme, surveiller la progression de la maladie et surveiller l'efficacité ou la réaction d'un sujet à un traitement particulier.

6. Un procédé de détermination du traitement approprié pour un sujet comprenant les opérations suivantes :
(a) la fourniture d'un échantillon biologique obtenu auprès d'un sujet,
(b) la détermination du niveau, ou la présence, de pHPRT dans l'échantillon biologique provenant dudit sujet, et
(c) l'utilisation des résultats de (b) de façon à déterminer la thérapie la plus appropriée.

7. Le procédé selon la Revendication 6 où la présence de pHPRT dans l'échantillon indique que le sujet doit être traité comme s'il était atteint de paludisme et que des médicaments antipaludiques doivent être administrés.

8. Le procédé selon la Revendication 6 ou 7 comprenant une opération complémentaire de comparaison du niveau de pHPRT déterminé à l'opération (b) à une ou plusieurs valeurs de référence prédéterminées avant l'opération (c).

9. Un procédé de détermination de la réaction d'un sujet atteint de paludisme à un traitement particulier comprenant les opérations suivantes :
(a) la fourniture d'un échantillon biologique obtenu auprès d'un sujet,
(b) la détermination du niveau de pHPRT dans l'échantillon biologique provenant dudit sujet, et
(c) la comparaison du niveau de pHPRT déterminé à l'opération (b) à une ou plusieurs valeurs de référence.

10. Le procédé selon la Revendication 9 où la valeur de référence est le niveau de pHPRT dans un échantillon antérieur provenant du même sujet.

11. Un procédé de diagnostic de paludisme chez un sujet comprenant :
i) l'obtention d'un échantillon biologique obtenu auprès d'un sujet,
ii) la détermination de la présence, et éventuellement du niveau, de PF10_0121 (hypoxanthine phosphoribosyltransférase, pHPRT) dans l'échantillon biologique, et
iii) le diagnostic de paludisme si pHPRT est présent dans l'échantillon.

12. Un médicament antipaludique destiné à une utilisation dans le traitement de paludisme chez un sujet, où le médicament antipaludique est destiné à être administré lorsque pHPRT est présent dans un échantillon obtenu auprès du sujet.

13. Le procédé ou le médicament antipaludique selon l'une quelconque ou plusieurs des Revendications précédentes où l'échantillon biologique est un échantillon de plasma.
